(19) Europäisches Patentamt
European Patent Office
Office européen des brevets

(11) EP 0 574 000 B1

(12) EUROPÄISCHE PATENTSCHRIFT

(45) Veröffentlichungstag und Bekanntmachung des Hinweises auf die Patenterteilung:
**27.08.1997 Patentblatt 1997/35**

(51) Int Cl.6: **G01N 33/547**, G01N 33/543, G01N 33/553

(21) Anmeldenummer: **93109319.9**

(22) Anmeldetag: **09.06.1993**

# (54) Selbst assemblierende Monoschicht mit kurzkettigen Linkern

Self-assembling mono-layer with short-chained linker

Monocouche auto-assemblante avec linker à chaîne courte

(84) Benannte Vertragsstaaten:
**AT BE CH DE DK ES FR GB GR IE IT LI LU NL PT SE**

(30) Priorität: **11.06.1992 DE 4219159**

(43) Veröffentlichungstag der Anmeldung:
**15.12.1993 Patentblatt 1993/50**

(73) Patentinhaber: **BOEHRINGER MANNHEIM GMBH**
**68298 Mannheim (DE)**

(72) Erfinder:
- **Guder, Hans-Joachim, Dr.**
  **D-8120 Weilheim (DE)**
- **Klein, Christian, Dr.**
  **D-8120 Weilheim (DE)**
- **Liley, Martha, Dr.**
  **London SW 13 9 JR (GB)**
- **Spinke, Jürgen**
  **D-6233 Kelkheim-Münster (DE)**
- **Knoll, Wolfgang, Prof. Dr.**
  **D-6500 Mainz (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**81635 München (DE)**

(56) Entgegenhaltungen:
**EP-A- 0 339 821** **EP-A- 0 410 280**
**WO-A-92/10757**

- **ANGEW. CHEM. Bd. 101, Nr. 4, 1989, Seiten 522 - 528 BAIN ET AL. 'Modeling organic surfaces with self-assembled monolayers'**
- **JOURNAL OF THE AMERICAN CHEMICAL SOCIETY Bd. 105, Juni 1983, Seiten 4481 - 4483 NUZZO ET AL. 'Adsorption of bifunctional organic disulfides on gold surfaces'**
- **JOURNAL OF THE AMERICAN CHEMICAL SOCIETY Bd. 112, 1990, Seiten 3239 - 3241 EBERSOLE ET AL. 'Spontaneously formed functionally active avidin monolayers on metal surfaces: ....'**
- **LANGMUIR Bd. 4, 1988, WASHINGTON DC Seiten 365 - 385 TROUGHTON ET AL. 'Monolayer films prepared by the spontaneous self-assembly .......'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft ein Verfahren zur Herstellung einer Bindematrix, die ein Trägermaterial und einen daran über Ankergruppen adsorbierten Festphasen-Reaktanden, der mit mindestens einem freien Reaktionspartner bindefähig ist, enthält, wobei dieser Festphasen-Reaktand eine verdünnte Bindeschicht auf der Oberfläche des Trägermaterials bildet.

Bei molekularen Erkennungsreaktionen handelt es sich um die feste und spezifische Bindung zweier Moleküle, die ohne die Ausbildung einer kovalenten Atombindung eintritt. Für eine praktische Handhabung sind insbesondere solche Reaktionen von Interesse, die an der Grenzfläche zwischen einem festen Trägermaterial und einer fluiden Umgebung ablaufen. Dazu wird die Oberfläche des festen Trägermaterials mit einer Fixierschicht belegt, die einen Festphasen-Reaktanden enthält. An dieser Fixierschicht laufen dann die eigentlichen Erkennungsreaktionen ab.

Ein Beispiel einer solchen Fixierschicht ist an polymerisiertes Albumin gebundenes Streptavidin, das wiederum gut an Kunststoffoberflächen adsorptiv bindet. Diese Festphase kann mittels Bindung an Biotin bzw. an biotinylierte Reaktanden für eine große Zahl von Immuntests verwendet werden. Diese auf Streptavidin/Polyalbumin basierende Bindematrix ist sehr gut für "große" Kunststoffoberflächen geeignet. Verkleinert man jedoch die beschichtete Oberfläche, so nimmt die Genauigkeit der Tests ab. Bei neuen Testsystemen - z.B. klassischer ELISA oder Bestimmung über optische oder elektrochemische Sensoren - besteht ein wachsender Bedarf an Miniaturisierung.

In Arbeiten von Blankenburg et al. (Biochemistry 28 (1989), 8214) und Ahlers et al. (Thin Solid Films 180 (1989) 93-99) werden Streptavidin-Monoschichten beschrieben, die auf einem Langmuir-Blodgett (LB)-Film basieren. Dazu werden zunächst Biotinlipidmonoschichten mit der komplizierten Technik der Filmwaage hergestellt, die anschließend mit einer Streptavidinlösung ca. 2 Stunden lang inkubiert werden müssen. Ein Nachteil dieser LB-Filme ist außerdem ihre begrenzte Stabilität, insbesondere gegenüber Austrocknen.

Eine weitere Möglichkeit zur Herstellung einer Fixierschicht auf einem Trägermaterial ist das sogenannte "Self-assembled monolayer" (SAM). So beschreiben Nuzzo und Allara (J.Am.Chem. Soc. 105 (1983), 4481-4483) die Adsorption von organischen Disulfiden auf Gold, die zu einer dichtgepackten Monoschicht führt. Die spontane Organisation solcher Monoschichten (daher die Bezeichnung SAM) beruht auf starken spezifischen Wechselwirkungen zwischen dem Trägermaterial und dem Adsorbat. Bain und Whitesides (Angew.Chem. 101 (1989), 522-528) beschreiben SAM, die durch Adsorption von langkettigen Thiolen an Goldoberflächen entstehen. So erhält man durch Inkubation einer Goldoberfläche mit Thiolen der Formel

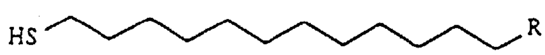

R = Alkyl, Vinyl, Halogen, Carbonsäure, Ester, Amid, Nitril, OH, Ether.

aus verdünnter organischer Lösung (z.B. 1 mmol/l) eine dichtgepackte Monoschicht. Diese Monoschichten sind im trockenen Zustand, in Wasser oder Ethanol bei Raumtemperatur über mehrere Monate stabil. Bei Erhitzen auf Temperaturen über 70°C tritt Desorption ein. Die Stabilität der Monoschicht steigt mit der Länge der aliphatischen Kette des Thiols. Auch gegen verdünnte Säuren (z.B. 1 N HCl) oder gegen verdünnte Laugen (z.B. 1 N NaOH) sind diese Monoschichten über einen gewissen Zeitraum (1 bis 7 Tage) stabil.

Die EP-A-0 339 821 offenbart Polymere für die Beschichtung von Metalloberflächen, die Thiolgruppen als Bindevermittler zum festen Trägermaterial und Aminogruppen enthalten, um einen geeigneten Festphasenreaktanden, z.B. Biotin und dann daran Streptavidin zu binden. Bei diesen Thiolgruppen-haltigen Polymeren läßt sich jedoch ebenfalls aufgrund ihrer polymeren Natur keine streng homogene Beschichtung erreichen.

Eine Arbeit von Ebersole et al. (J. Am. Chem. Soc. 122 (1990), 3239-3241) offenbart funktionell aktive Monoschichten von Avidin und Streptavidin durch direkte Adsorption dieser Proteine an Gold- und Silberoberflächen. Dabei entsteht eine dichtgepackte Streptavidin-Bindephase, die trotz einer relativ hohen Inkubationszeit von 20 Minuten mit einem biotinylierten Bindepartner nur eine sehr unvollständige Belegung mit diesem Bindepartner ergibt.

Zusammenfassend ist zum Stand der Technik festzustellen, daß die dort beschriebenen Bindephasen auf Monoschichtbasis entweder langsam oder nur mit einer geringen Belegung einen freien Reaktanden binden können. Somit bestand ein großes Bedürfnis, diese Nachteile des Standes der Technik mindestens teilweise zu beseitigen. Insbesondere sollte eine möglichst mikroskopisch homogene Universalbindephase bereitgestellt werden, die in möglichst kurzer Zeit eine möglichst große Menge eines freien Reaktanden binden kann.

Hierzu wird in DE 40 39 677 und W092/10757, die nach dem Prioritätstag der vorliegenden Anmeldung veröffentlicht wurden, eine Bindematrix vorgeschlagen, enthaltend ein Trägermaterial und einen daran über Ankergruppen adsorbierten Festphasen-Reaktanden, der mit mindestens einem freien Reaktionspartner bindefähig ist, wobei der Festphasen-Reaktand eine verdünnte und im wesentlichen lateral homogene Bindeschicht auf der Oberfläche des Trägermaterials bildet. Der Festphasen-Reaktand kann mit der Ankergruppe über ein Spacermolekül verknüpft sein, wobei sich überdies zwischen dem Spacermolekül und dem Festphasen-Reaktanden eine hydrophile Linkergruppe, z.B. Diaminodioxooctan (DADOO), befinden kann.

Die Herstellung dieser Bindematrix erfolgte bisher so, daß man eine Lösung des Thiols in einem geeigneten organischen Lösungsmittel, z.B. Chloroform, Alkohol oder Mischungen aus beiden, herstellte und damit die Oberfläche des Trägermaterials, vorzugsweise Gold, unter geeigneten Bedingungen benetzte. Die Verwendung von organischen Lösungsmitteln ist jedoch in der großtechnischen Produktion mit Nachteilen behaftet. Zudem können diese Lösungsmittel die biologischen Eigenschaften des Festphasen-Reaktanden und des Bindepartners beeinflussen.

In einer Ausführungsform von DE 40 39 677 wird eine Bindematrix offenbart, die neben den mit Festphasen-Reaktanden verknüpften Spacermolekülen noch Spacermoleküle enthält, die mit Ankergruppen versehen sind, aber nicht mit Festphasen-Reaktanden verknüpft sind. Auf diese Weise kann man eine optimal gepackte Schicht des Festphasen-Reaktanden für die Anknüpfung eines freien Reaktionspartners und damit eine dicht gepackte Bindematrix erhalten. Ein Beispiel für ein geeignetes Spacermolekül ohne Festphasen-Reaktand (auch als Verdünner oder Verdünnungsmolekül bezeichnet) ist z.B. Mercaptoundecanol, d.h. eine langkettige lipophile Substanz.

Überraschenderweise wurde festgestellt, daß mit Festphasen-Reaktanden verknüpfte kurzkettige Spacermoleküle in Kombination mit hydrophilen Verdünnern aus wäßrigen Lösungen in einem self-assembly-Prozeß Monoschichten bilden, die als universelle Bindematrix noch vorteilhaftere Eigenschaften aufweisen. Die Verwendung derartiger hydrophiler Komponenten besitzt den Vorteil, daß man die Belegung des Trägermaterials aus Wasser oder einer wäßrigen Reaktionslösung vornehmen kann und dadurch keine Löslichkeitsprobleme mit dem Aufbau der Bindematrix hat, wenn der Festphasen-Reaktand stark hydrophile Eigenschaften besitzt. Ein weiterer Vorteil der Verwendung von wäßrigen Lösungsmitteln besteht darin, daß keine zusätzlichen Lösungsvermittler (z.B. Detergenzien) vorhanden sein müssen. Derartige Lösungsvermittler können nämlich den homogenen Aufbau der Bindematrix sowie die biologischen Eigenschaften des Festphasen-Reaktanden und des Bindepartners stören.

Gegenstand der vorliegenden Erfindung ist somit ein Verfahren zur Herstellung einer Bindematrix, das ein Trägermaterial und einen daran über Ankergruppen adsorbierten Festphasen-Reaktanden, der mit mindestens einem freien Reaktionspartner bindefähig ist, enthält, wobei der Festphasen-Reaktand eine verdünnte und im wesentlichen lateral homogene Bindeschicht auf der Oberfläche des Trägermaterials bildet, welches dadurch gekennzeichnet ist, daß man das Trägermaterial mit einer wäßrigen Reaktionslösung inkubiert, die einen über ein kurzkettiges Spacermolekül, welches eine Alkylengruppe der Formel $(CH_2)_n$ mit n = 1 bis 6 enthält, mit der Ankergruppe verknüpften Festphasen-Reaktanden und mindestens ein hydrophiles Verdünnungsmolekül enthält, welches ein mit Ankergruppen versehenes Molekül ist, an das kein Festphasen-Reaktand gebunden ist.

Die wäßrige Reaktionslösung ist Wasser oder ein wäßriges Puffersystem, das vorzugsweise weniger als 20 % (v/v), besonders bevorzugt weniger als 1 % (v/v) organische Lösungsmittel enthält. Am meisten bevorzugt enthält das wäßrige Puffersystem keine zusätzlichen Lösungsvermittler wie organische Lösungsmittel oder Detergenzien.

Das Trägermaterial der erfindungsgemäßen Bindematrix kann eine Metall- oder Metalloxidoberfläche aufweisen. Vorzugsweise besitzt das Trägermaterial eine Metalloberfläche, besonders bevorzugt eine Edelmetalloberfläche. Die Herstellung eines Trägermaterials mit einer Goldoberfläche geschieht beispielsweise durch Bedampfen von Glas mit Chrom als Haftvermittler, wobei eine ca. 0,1 bis 10 nm dicke Schicht entsteht. Diese Chromschicht wird anschließend mit Gold bedampft, wobei eine Goldschicht entsteht, welche die Oberfläche des Trägermaterials für eine erfindungsgemäße Bindematrix darstellt. Diese Goldschicht ist zweckmäßig zwischen ca. 10 und 100 nm dick, wenn die Bindematrix im Rahmen der Oberflächenplasmonenresonanz verwendet wird. Bei anderen Anwendungen, z.B. als elektrochemischer Sensor, kann die Bindematrix auch dicker gewählt werden.

Die Adsorption des Festphasen-Reaktanden an die Oberfläche des Trägermaterials wird durch Ankergruppen vermittelt. Die Art der Ankergruppen hängt von der jeweiligen Oberfläche des Trägermaterials ab. Als Ankergruppen für ein Trägermaterial mit einer Metalloberfläche sind Thiol-, Disulfid- oder Phosphingruppen geeignet. So sind z.B. Thiol- oder Disulfidgruppen als Ankergruppen für Gold- oder Silberoberflächen und Phosphingruppen für eine Palladiumoberfläche besonders geeignet. Weist das Trägermaterial eine Metalloxidoberfläche (z.B. $Al_2O_3$) auf, so ist als Ankergruppe eine Carboxylat- oder Sulfonatgruppe geeignet.

Die Ankergruppe für die Adsorption an die Festphase ist nicht direkt am Festphasen-Reaktanden selbst angebracht, sondern ist über ein kurzkettiges Spacermolekül, vorzugsweise über ein flexibles Spacermolekül mit dem Festphasen-Reaktanden verknüpft. Als kurzkettiges Spacermolekül gilt im Rahmen der Erfindung eine Alkylengruppe der Formel $(CH_2)_n$, worin n eine natürliche Zahl von 1 bis 6, vorzugsweise 1 bis 4, besonders bevorzugt 1 bis 3 darstellt. Auf seiner einen Seite enthält das Spacermolekül die Ankergruppe (z.B. die Thiol- oder Disulfidgruppe), die zur Adsorption an die Oberfläche des Trägermaterials geeignet ist. Auf seiner anderen Seite, d.h. vom Trägermaterial wegstehend enthält das Spacermolekül eine oder mehrere Verknüpfungsgruppierungen, über die der Festphasen-Reaktand oder eine Komponente davon mit dem Spacermolekül verknüpft ist. Bei diesen Verknüpfungsgruppierungen kann es sich z.B. um eine Amino- oder Hydroxylfunktion handeln, die z.B. mit einer Carboxylfunktion des Festphasen-Reaktanden unter Bildung einer Ester- oder Amidgruppe verknüpft ist. Das Spacermolekül kann jedoch auch als Verknüpfungsgruppe eine Carboxylfunktion enthalten, die dann wiederum mit einer reaktiven Amino- oder Hydroxylfunktion des Festphasen-Reaktanden verknüpft ist. Bei der Auswahl des Spacermoleküls ist eine geringe Kettenlänge wesentlich, da bei einem zu großen hydrophoben Rest der Komplex aus Festphasen-Reaktand und Spacer nicht mehr ausreichend wasserlöslich ist.

Zunächst soll eine nicht erfindungsgemäße, dichtgepackte Monoschicht eines Festphasen-Reaktanden beschrieben werden, welche auch nicht aus wäßriger Lösung herstellbar ist. Eine solche Schicht ist erhältlich, wenn man ein Lipid gemäß der Arbeit von Bain und Whitesides (Angew. Chem. 101 (1989), 522-528), das eine Hydroxyl- oder Aminoendgruppe aufweist, mit einem aktivierten Biotinderivat umsetzt, wobei unter Verwendung von 11-Hydroxy-undekan-1-thiol als Ausgangsmaterial ein biotinyliertes Alkanthiol mit folgender Formel entsteht:

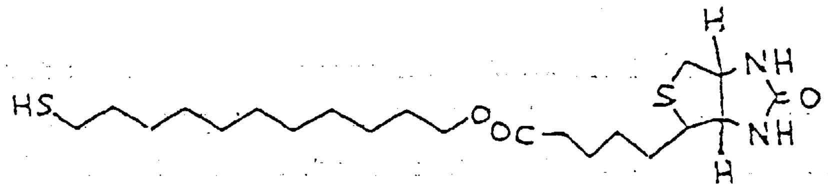

Bei Adsorption dieses Alkanthiols an ein Trägermaterial mit Goldoberfläche bis zur Sättigung entsteht gemäß Dickenmessung eine dicht gepackte Monoschicht mit einer Belegung von 100 % bezogen auf Biotin. Auf diese Weise erhält man eine starre Bindematrix, die als solche nicht erfindungsgemäß ist und auch eine nur geringe Bindekapazität für einen freien Reaktionspartner (in diesem Falle Streptavidin) besitzt.

Im Gegensatz dazu ist die Herstellung einer erfindungsgemäßen Bindematrix durch Verwendung eines Spacermoleküls möglich, das mit zwei oder mehreren Molekülen, vorzugsweise 2 Molekülen des Festphasen-Reaktanden gleichzeitig verknüpft ist. Ein Beispiel für ein solches Spacermolekül ist Cystamin, das als Ankergruppe eine Disulfidgruppe und als Verknüpfungsgruppierung zwei Aminofunktionen enthält und daher mit zwei Molekülen eines aktivierten Biotins verknüpft werden kann, wobei eine biotinylierte Verbindung mit folgender Formel entsteht:

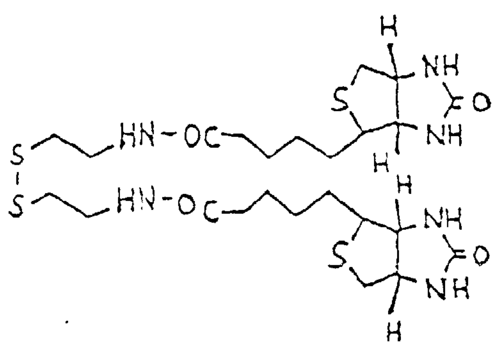

Diese biotinylierte Verbindung bildet bei Adsorption an eine Goldoberfläche eine erfindungsgemäße Bindematrix mit einem Belegungsgrad von 30 % bezogen auf Biotin aus, die einen freien Reaktionspartner (Streptavidin) mit hoher Affinität zu einem dichten Film binden kann.

Vorzugsweise befindet sich eine hydrophile Linkergruppe zwischen dem Spacermolekül und dem Festphasen-Reaktanden. Dieser Linker ist insbesondere ein geradkettiges Molekül mit einer Kettenlänge von 4 bis 15 Atomen, wobei die Kette des Linkers vorzugsweise aus C-Atomen und Heteroatomen (vorzugsweise N- und O-Atomen) zusammengesetzt ist. Besonders bevorzugt ist dabei eine Linkergruppe, die eine oder mehrere hydrophile Ethylenoxideinheiten, vorzugsweise zwischen 1 und 5 enthält. Besonders bevorzugt wird die hydrophile Linkergruppe durch ein Amin- oder Hydroxyl-terminiertes Polyethylenoxid gebildet.

Zwischen dem hydrophilen Linker und dem Festphasen-Reaktanden kann vorzugsweise noch ein weiteres Spacermolekül eingebaut werden, welches aus einer Alkylengruppe der Formel $(CH_2)_n$ und einer Verknüpfungsgruppierung besteht, worin n eine natürliche Zahl von 2 bis 12, vorzugsweise 2 bis 8 ist.

Als besonders geeigneter Linker für den Festphasen-Reaktanden hat sich 1,8-Diamino-3,6-dioxaoctan erwiesen. So entsteht durch den Einbau von 1,8-Diamino-3,6-dioxaoctan zwischen ein $C_3$-Thiolspacermolekül und ein Biotin-Spacer-Molekül eine biotinylierte Verbindung der folgenden Formel:

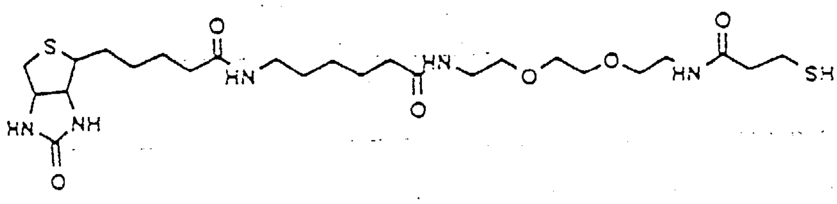

Die wäßrige Reaktionslösung zur Herstellung einer Bindematrix enthält neben dem oben beschriebenen Festphasen-Reaktanden noch ein hydrophiles Verdünnungsmolekül, d.h. ein mit Ankergruppen versehenes Molekül, an das <u>kein</u> Festphasen-Reaktand gebunden ist. Geeignete Verdünnungsmoleküle enthalten eine Ankergruppe und einen Spacerbestandteil sowie gegebenenfalls eine Linkergruppe, wobei die Anzahl der C-Atome des Spacermoleküls vorzugsweise 1 bis 6, besonders bevorzugt 1 bis 4 und am meisten bevorzugt 1 bis 3 beträgt. Auch hier ist bei der Auswahl des Spacermoleküls eine geringe Kettenlänge bevorzugt, da bei einem zu großen hydrophoben Rest das Verdünnungsmolekül nicht mehr ausreichend wasserlöslich ist.

Anstelle des Festphasen-Reaktanden befindet sich vorzugsweise an dem von der Ankergruppe entfernten Ende des Verdünnungsmoleküls eine hydrophile Funktion, wie z. B. eine Hydroxylgruppe, eine Carbonsäuregruppe, eine Carbonsäureethylester- oder Methylestergruppe, eine Carbonsäureamidgruppe, eine mit 1 oder 2 Methyl- oder Ethylgruppen substitutierte Carbonsäureamidgruppe, eine Sulfonsäuregruppe oder eine Sulfonamidgruppe. Ebenso ist es bevorzugt, an das von der Ankergruppe entfernte Ende des Verdünnungsmoleküls einen hydrophilen Linker (gemäß obiger Definition) oder einen Teil eines hydrophilen Linkers zu binden. Folglich enthält ein bevorzugtes Verdünnungsmolekül auf der einen Seite des Spacerbestandteils eine mit dem Trägermaterial reaktive Ankergruppe und auf der anderen Seite eine hydrophile Endgruppe.

Der Gesamtcharakter des Verdünnungsmoleküls muß so hydrophil sein, daß es in Wasser oder einem wäßrigen Puffer löslich ist. Weiterhin muß das Verknüpfungsmolekül zur spontanen Anlagerung an das Trägermaterial in der Lage sein. Überraschenderweise wurde gefunden, daß das Verdünnungsmolekül eine erheblich geringere Kettenlänge als die des mit dem Festphasen-Reaktanden gekoppelten Spacer/Linkers aufweisen darf. Beispielsweise ergibt ein Verdünnungsmolekül, das weniger als 50 % der Kettenlänge des an den Festphasen-Reaktanden gekoppelten Spacer/Linkers sogar noch bessere Resultate als ein Verdünnungsmolekül mit einer Kettenlänge, die der des Spacer/Linkers am Festphasen-Reaktanden entspricht.

Das Verdünnungsmolekül ist somit vorzugsweise eine neue Verbindung der allgemeinen Formeln

$$X^1 - S - S - X^2 \text{ oder } HS - X^3$$

worin $X^1$, $X^2$ und $X^3$ jeweils

$$-(CH_2)_n-\overset{O}{\overset{\|}{C}}-NH-L-Y$$

bedeuten,
worin

- n eine natürliche Zahl von 1 bis 6 bedeutet,
- L eine hydrophile Linkergruppe mit einer Kettenlänge von 4 bis 15 Atomen ist und
- Y eine hydrophile Endgruppe, vorzugsweise $-NH_2$, -OH, -COOH oder $-SO_3H$ ist.

Zum Aufbau einer erfindungsgemäßen Bindematrix kann man aber auch ein Gemisch aus mehreren der oben genannten Verbindungen verwenden.

Ein besonders bevorzugtes Beispiel eines kurzen hydrophilen Verdünnungsmoleküls ist 2-Mercaptopropionsäure-[2-(2-hydroxyethoxy)]-ethylamid.

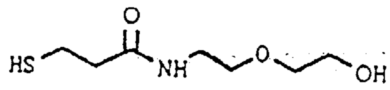

In einer weiteren Ausführungsform der Erfindung können ein Spacer mit Festphasen-Reaktand und ein Spacer ohne Festphasen-Reaktand über eine kovalente Bindung verknüpft sein. Bei Verwendung von Gold- oder Silberoberflächen erfolgt diese Verknüpfung vorzugsweise über eine Disulfidbrücke.

In solchen gemischten Monoschichten, die aus Verdünnungsmolekülen (Spacermolekülen ohne Festphasen-Reaktand) und aus Spacermolekülen mit Festphasen-Reaktand bestehen, beträgt der Anteil der Spacermoleküle mit Festphasen-Reaktand zweckmäßig 0,1 - 90 mol-%, vorzugsweise 0,5 - 70 mol-% und besonders bevorzugt 1 - 40 mol-%.

Der Festphasen-Reaktand ist vorzugsweise Biotinanaloge Biotin oder Moleküle wie Desthiobiotin, Iminobiotin oder HABA (4-Hydroxy-phenyl-azo-benzoesäure), die ebenfalls mit Streptavidin, Avidin oder Derivaten davon reagieren.

Ein besonderer Aspekt des erfindungsgemäßen Bindefilms besteht in der Möglichkeit, als Festphasen-Reaktand Biotinderivate einzusetzen, die wie Desthiobiotin eine wesentlich geringere Bindekraft gegenüber Streptavidin aufweisen. Bei Verwendung dieser Festphasen-Reaktanden ist es möglich, gebundenes Streptavidin durch Zugabe einer Lösung, die normales Biotin enthält, zu verdrängen, so daß der Universalbindefilm auf diese Weise wieder verwendbar wird.

Weitere Beispiele für geeignete Festphasen-Reaktanden sind jedoch auch mit einem Antikörper bindefähige Antigene oder Haptene. In diesem Fall ist der Festphasen-Reaktand vorzugsweise ein Hapten mit einem Molekulargewicht von 100 bis 1200. Geeignet sind beispielsweise Steroide (wie z.B. Digoxin, Digoxigenin, Cortisol, Oestriol, Oestradiol, Theophyllin, Diphenylhydantoin, Testosterol, Gallensäuren, Progesteron und Aldosteron); kurzkettige Peptide (wie z.B. Argipressin, Oxytocin und Bradykinin); Fluorescein und seine Derivate; $T_3$, $T_4$, Aflatoxin, Atrazin, Pflanzenhormone wie z.B. Gibberilline; und Alkaloide (wie z.B. Reserpin und Ajmalicin).

Besonders bevorzugt werden als Hapten Biotin und Biotinderivate, Digoxin, Digoxigenin, Fluorescein und Derivate sowie Theophyllin eingesetzt.

Andererseits kann der Festphasen-Reaktand auch aus mehreren Komponenten bestehen. Darunter ist insbesondere zu verstehen, daß eine innere Komponente des Festphasen-Reaktanden kovalent mit einem Spacermolekül verknüpft ist und nicht kovalent an die äußere Komponente des Festphasen-Reaktanden gebunden ist. Dabei ist dann die äußere Komponente des Festphasen-Reaktanden zur Bindung eines freien Reaktionspartners in der Lage. Beispielsweise kann es sich bei der inneren Komponente um Biotin und bei der äußeren Komponente um Streptavidin handeln. Eine solche Bindematrix ist wiederum in der Lage, biotinylierte Reaktionspartner (z.B. biotinylierte Antikörper) aus einer Lösung zu binden, da Streptavidin vier Bindestellen für Biotin besitzt, von denen mindestens zwei noch frei sind.

Eine Bindeschicht, die einen aus zwei Komponenten bestehenden Festphasen-Reaktanden enthält, ist dann eine erfindungsgemäße Bindematrix, wenn die äußere, d.h. die mit einem freien Reaktionspartner bindefähige Komponente des Festphasen-Reaktanden (d.h. im speziellen Fall Streptavidin) eine verdünnte Schicht an der Oberfläche der Bindematrix bildet.

Dieses erfindungsgemäße Prinzip des verdünnten bindefähigen Festphasen-Reaktanden läßt sich von der Biotin-Streptavidin-Bindung auch auf andere Bindepaare, also z.B. Antikörper-Antigen etc. erweitern.

Der Belegungsgrad des Festphasen-Reaktanden an der Oberfläche der Bindematrix ist durch eine Dickenmessung der Bindeschicht bestimmbar. Dabei nimmt die gemessene Schichtdicke mit dem Belegungsgrad der Bindeschicht ab.

Aufgrund der unterschiedlichen Natur der erfindungsgemäßen Bindematrizes sind auch die Herstellungsverfahren im Detail unterschiedlich. Eine bevorzugte Variante dieser Herstellungsverfahren ist in den Ausführungsbeispielen dargestellt. Allgemein beinhaltet das erfindungsgemäße Verfahren die Inkubation des Trägermaterials mit einer wäßrigen Reaktionslösung, in der die Moleküle vorliegen, welche die Bindeschicht der erfindungsgemäßen Bindematrix bilden. Diese Moleküle enthalten an gegenüberliegenden Seiten die Ankergruppe und den Festphasen-Reaktanden, wobei - wie oben ausgeführt - nicht alle Moleküle der Bindeschicht mit einem Festphasen-Reaktanden verknüpft sind. Vorzugsweise ist der Festphasen-Reaktand über ein Spacermolekül mit der Ankergruppe verknüpft. Die Anlagerung der Ankergruppen an das Trägermaterial aus der Lösung unter Bildung der erfindungsgemäßen Bindematrix ist ein spontaner Prozeß.

Die Inkubation des Trägermaterials mit der Reaktionslösung zur Herstellung der ersten Bindeschicht findet vorzugsweise unter Schutzgas, in Wasser oder einem wäßrigen Puffer und ohne Zusatz von störenden Substanzen, wie organischen Lösungsmitteln und Detergenzien statt.

Gegebenenfalls kann in einem zweiten Schritt durch Inkubation mit einer zweiten Reaktionslösung eine weitere Substanz angelagert werden, insbesondere wenn der Festphasen-Reaktand aus mehreren, nicht kovalent miteinander

verbundenen Komponenten besteht. Zum Aufbringen der zweiten und ggf. weiteren Schichten sind die Reaktionsbedingungen nicht kritisch, so daß ohne Schutzgas gearbeitet werden kann.

Ein weiterer Gegenstand der vorliegenden Erfindung ist somit ein Verfahren, wobei man nach Adsorption des mit Ankergruppen versehenen Festphasen-Reaktanden an das Trägermaterial die resultierende Bindematrix mit einer oder mehreren weiteren, mit der Bindematrix bindefähigen Substanzen inkubiert, um einen aus mehreren, nicht-kovalent verknüpften Komponenten bestehenden Festphasen-Reaktanden zu erzeugen.

Die nach dem erfindungsgemäßen Verfahren hergestellte laterale Bindeschicht ist mikroskopisch homogen, wie z.B. durch die Surface plasmon microscopy nachweisbar (B. Rothenhäusler und W. Knoll, Surface Plasmon Microscopy, Nature, Vol. 332, Nr. 6165, S. 615-617 (1988); W. Hickel, B. Rothenhäusler und W. Knoll, "Surface Plasmon Microscopic Characterisation of external surfaces", J.Appl.Phys. (1989), S. 4832-4836; W. Hickel, W. Knoll "Surface Plasmon optical characterisation of lipid monolayers at 5 μm lateral resolution", J.Appl.Phys. 67 (1990), S. 3572 ff.). Bei einer Auflösung von 5 μm sind keine Dickenunterschiede meßbar.

Weiterhin betrifft die vorliegende Erfindung ein Verfahren zur Bestimmung eines Analyten in einer Probelösung mittels einer spezifischen Bindungsreaktion zwischen mindestens zwei bioaffinen Reaktanden, von denen einer an eine Festphase gekuppelt vorliegt und der oder die anderen Partner frei sind, wobei man einen Festphasen-Reaktanden verwendet, der Bestandteil einer erfindungsgemäßen Bindematrix ist.

Bei einem derartigen Verfahren kann der Nachweis der Bindung des freien Reaktionspartners an den Festphasenreaktanden dadurch ermöglicht werden, daß der freie Bindungspartner eine Markierungsgruppe trägt. Gebräuchlich ist insbesondere die Markierung mit einem Enzym oder mit einem fluoreszierenden oder lumineszierenden Bestandteil. Die dadurch ermöglichte indirekte optische Beobachtung der Bindung erlaubt einen genauen quantitativen Nachweis.

Grundsätzlich kann die Bindung optisch, elektrochemisch, aber auch über die Wärmetönung oder die Massenbildung bestimmt werden. Für elektrochemische Techniken kommen insbesondere potentiometrische und amperometrische Methoden in Frage, wie sie beispielsweise in "Biosensors", Turner, Karube, Wilson (eds.), Oxford Press, 1987 oder Bergveld, Biosensors & Bioelectronics 6, 55-72 (1991) beschrieben sind. Bestimmungen über die elektrische Leitfähigkeit oder Kapazitätsänderung sind als elektrochemische Techniken ebenfalls möglich.

Vorzugsweise erfolgt jedoch der Nachweis der Bindung durch optische, insbesondere reflexionsoptische Techniken, bei der die Zunahme der Schichtdicke einer extrem dünnen Schicht mit dem trägerfixierten Reaktanden durch Bindung des freien Bindungspartners beobachtet werden kann. Ein Überblick über diese Techniken wird gegeben in Sadowski: "Review of optical methods in immunosensing", SPIE, Vol. 954 Optical testing and Metrology II (1988), 413-419.

Ein besonders bevorzugtes reflexionsoptisches Verfahren ist der Nachweis der Bindung durch Oberflächenplasmonenresonanz. Bei diesem Verfahren besteht das Analyseelement aus einem durchsichtigen dielektrischen Material, auf dem in sehr geringer Schichtdicke eine metallisch leitende Schicht aufgebracht ist, welche den Festphasen-Reaktanden trägt. Dieses Analyselement wird vielfach auch als optischer Immunsensor bezeichnet. Beispiele für solche optischen Immunsensoren sind in der EP-A 0 112 721, der EP-A 0 276 142 und in der EP-A 0 254 575 beschrieben. Besonders bevorzugt für den quantitativen Nachweis der Bindung an die Festphase ist jedoch ein Immunsensor, der detailliert in der DE 40 24 476 offenbart wird.

Wie oben beschrieben, kann ein Festphasen-Reaktand verwendet werden, der gegenüber dem freien Reaktionspartner eine vergleichsweise geringe Bindekraft aufweist. In diesem Falle ist es möglich, den an die Festphase gekoppelten Reaktionspartner durch Zugabe eines weiteren freien Reaktanden mit höherer Bindekraft abzulösen und somit die Bindematrix zu regenerieren.

Besonders bevorzugt kann man für eine regenerierbare Bindematrix Biotinderivate, wie etwa Desthiobiotin und Iminobiotin, aufgrund der geringeren Bindeaffinität an Streptavidin als Biotin als Festphasenreaktanden verwenden. (Die Bindekonstante von Desthiobiotin/Streptavidin ist $10^{12}$, während die Bindungskonstante von Biotin/Streptavidin $10^{15}$ ist). Wenn man daher einen Festphasenreaktanden mit geringerer Affinität verwendet, ist es möglich, gebundenes Streptavidin durch Zusatz einer Lösung zu entfernen, die einen höher affinen Reaktanden (z.B. Biotin) enthält, so daß auf diese Weise der Universalbindefilm mehrfach verwendet werden kann.

Für die Durchführung von Immunoassays unter Verwendung einer regenerierbaren Desthiobiotin- oder Iminobiotin-Bindephase ist es günstig, desthiobiotinylierte oder iminobiotinylierte Antikörper oder Antikörperfragmente oder desthiobiotinylierte oder iminobiotinylierte (Poly-)Haptene zu verwenden.

Ein weiterer Gegenstand der vorliegenden Erfindung ist somit ein Verfahren, welches dadurch gekennzeichnet ist, daß man die Bindematrix nach Durchführung einer Analytenbestimmung regeneriert, indem der an den Festphasen-Reaktanden gebundene freie Reaktionspartner durch Zugabe eines weiteren freien Reaktanden von der Bindematrix abgelöst wird. Dabei ist es bevorzugt, daß der weitere freie Reaktand eine höhere Affinität gegenüber dem Reaktionspartner als der Festphasen-Reaktand besitzt. Beispiele für geeignete Paare aus Festphasen-Reaktand und freiem Reaktand sind etwa Desthiobiotin und Biotin, oder entsprechend zwei Haptene, die sich chemisch nur gering unterscheiden und somit gegenüber einem Antikörper eine etwas unterschiedliche Bindekraft besitzen.

Die erfindungsgemäß hergestellte Bindematrix kann somit auch als regenerierbare Schicht verwendet werden.

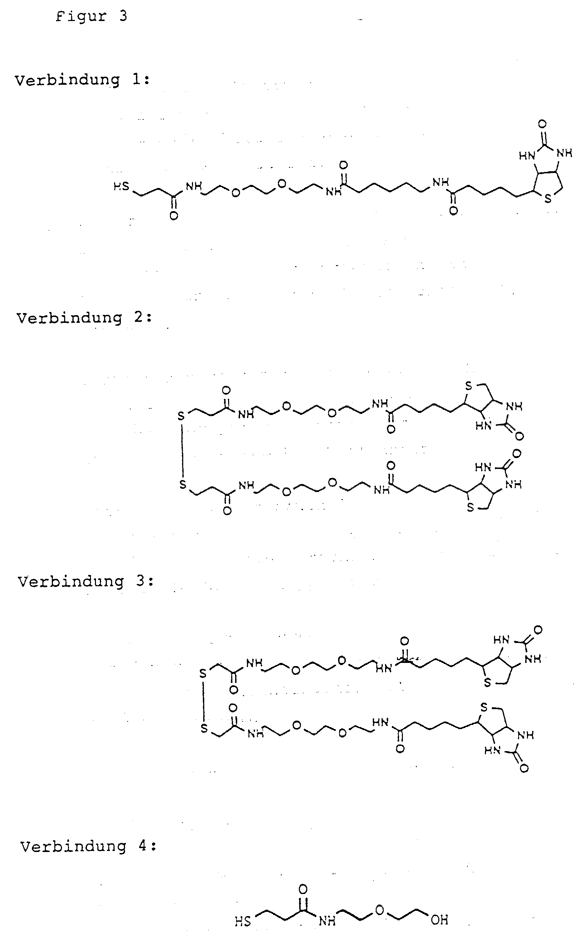

Die vorliegende Erfindung soll im weiteren durch die folgenden Beispiele in Verbindung mit den Figuren 1 bis 3 verdeutlicht werden.

Fig. 1 ist die schematische Darstellung einer Meßeinrichtung, mit der die Bindung eines freien Reaktionspartners an die Festphase bestimmt werden kann.

Fig. 2 zeigt die Dicke von Bindematrices, hergestellt durch die Kombination einer Komponente mit Festphasen-Reaktanden und eines Verdünnungsmoleküls sowie die Dicke des daran gebundenen Streptavidins (Beispiel 12):

Kurve 1: Dicke der Biotinmonolayer
Kurve 2: Dickenzuwachs durch Streptavidin in Abhängigkeit vom Molenbruch x an Biotin

$$x = \frac{C_{\text{Biotinverbindung 1}}}{C_{\text{Biotinverbindung 1}} + \text{Verdünnungsmoleküle}} : 2$$

C: molare Konzentration

Fig. 3 zeigt erfindungsgemäß verwendete Verbindungen, wobei Verbindung 1 die in Beispiel 7 hergestellte Biotinverbindung ist, Verbindung 2 die in Beispiel 9 hergestellte Biotinverbindung ist, Verbindung 3 die in Beispiel 11 hergestellte Biotinverbindung ist und Verbindung 4 das in Beispiel 12b hergestellte Verdünnungsmolekül ist.

Beispiel 1

Synthese von Bisbiotinoylcystamin

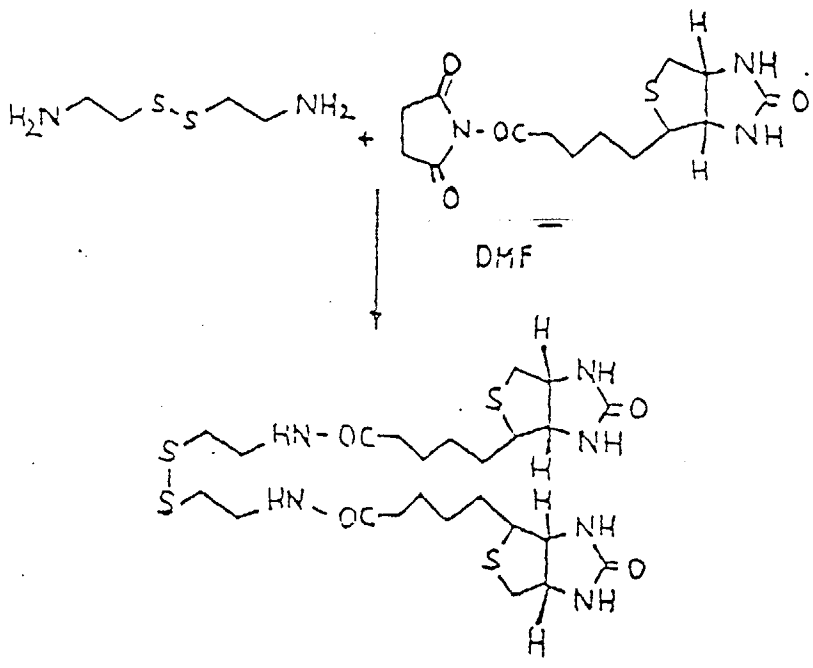


Biotin-N-hydroxysuccinimidester wurde zu einer Lösung von Cystaminiumdichlorid und Triethylamin in Dimethylformamid (DMF) gegeben. Die Reaktionsmischung wurde über Nacht bei Raumtemperatur gerührt. Nach beendeter Reaktion wurde der entstandene Niederschlag abfiltriert, an der Ölpumpe getrocknet und aus Aceton umkristallisiert. Die Zielverbindung wurde in 40 %iger Ausbeute erhalten.

Beispiel 2:

**Bestimmung der Schichtdicken von erfindungsgemäßen Monoschichten**

Figur 1 zeigt schematisch eine Meßeinrichtung zur reflexionsoptischen Bestimmung der Bindung des freien Reaktionspartners an die Festphase.

Eingebaut in einen modifizierten Kretschmann Aufbau (Fig. 1) kann die beschichtete Probe sowohl gegen Luft als auch gegen wäßrige Medien charakterisiert werden.

Diese reflexionsoptische Meßeinrichtung enthält einen Laser 10. Der von dem Laser ausgehende Primärstrahl 11 fällt unter einem Winkel θ zur Flächennormalen 17 des Testbereichs 20 ein. Das reflektierte Licht wird von einer Sammellinse 12 auf die in der Bildebene angeordnete Diode 13 abgebildet.

Die Meßeinrichtung enthält weiterhin ein Prisma 16 in Kretschmann-Anordnung und eine Flußküvette 14 mit Zutritts- und Ausgangsöffnungen 15 für die Testlösung.

Der Testbereich 20 besteht aus einem Prisma 16 in Kretschmann-Anordnung, einer dielektrischen, optisch transparenten Trägerschicht 22 und einer dünnen Metallschicht 23a, 23b, die auf die Trägerschicht 22 aufgedampft ist. Die dünne Schicht der Indexflüssigkeit 21 verbindet das Prisma 16 ohneoptische Brechung mit der optisch transparenten Trägerschicht 22, da sie den gleichen Brechungsindex wie diese beiden Teile besitzt. Im vorliegenden Falle stellt 23a die oben erwähnte Chromschicht und 23b die die aufgedampfte Goldschicht dar. 25 stellt das Spacermolekül dar, welches über Ankergruppen die Bindung des Festphasen-Reaktanden 26 an die Goldoberfläche vermittelt. 27 stellt den freien Reaktionspartner dar, der mit dem Festphasenreaktanden bindefähig ist und in der Testphase 28 vorliegt.

Mit Hilfe der Fresnel'schen Gleichungen läßt sich die Reflexion an Grenzflächen berechnen und an die PSP-Spektroskopie Daten anfitten, wobei man für jede Schicht eine Angabe zur "optischen Dicke" (= n x d, mit n = Brechnungsindex und d = Dicke) erhält.

Beispiel 3

**Synthese von 1-tert.Butyloxycarbonyl-1,8-diamino-dioxaoctan, (mono-BOC-DADOO)**

Eine Lösung von 142 g (1 mol) 1,8-Diamino-3,6-dioxaoctan (DADOO) in 900 ml Dioxan/Wasser (1/1 v/v) wird unter rühren langsam mit einer Lösung von 109 g (0,5 mol) Di-tert.butyldicarbonat in 450 ml Dioxan versetzt. Nach der Zugabe wird das Gemisch noch 1,5 h bei 20°C gerührt, anschließend das Lösungsmittel abdestilliert und der Rückstand in 1 l Ethylacetat/Wasser (1/1 v/v) aufgenommen. Nach dem Abtrennen der wäßrigen Phase extrahiert man die organische Phase zweimal mit je 100 ml 0,1 N HCl. Die wäßrigen Phasen werden vereinigt, der pH-Wert mit verdünnter Natronlauge auf pH 9 bis 10 gestellt und die Lösung einer Flüssig-Flüssig-Extraktion im Perforator unterworfen. Nach 8-stündigem Extrahieren mit 750 ml Ethylacetat wird das Lösungsmittel entfernt und der Rückstand am Hochvakuum getrocknet.
Ausbeute: 32 g (26 %)
DC: Kieselgel 60,
Eluens Butylacetat/Wasser/Ammoniumhydroxid = 30/15/5,
RF = 0,45

Beispiel 4

**Synthese von 1-(Biotin-aminocapronsäure)-(1,8-diamino-4,6-dioxaoctan)-amid, (Biotin-X-DADOO)**

Eine Lösung von 0,9 g (2 mmol) D-Biotinoyl-aminocapronsäure N-hydroxysuccinimidester (Boehringer Mannheim, Best.Nr. 1003933) und 0,5 g (2 mmol) mono-BOC-DADOO in 10 ml Dioxan und 10 ml 0,1 mol/l Kaliumphosphatpuffer pH 8,5 werden ca. 2 Stunden bei 20°C gerührt. Nach beendeter Reaktion (DC-Kontrolle: Kieselgel 60, Eluens Ethylacetat/Methanol = 3/7, RF = 0,6) wird das Lösungsmittel im Vakuum abgedampft und der Rückstand mit 1 ml Trifluoressigsäure versetzt. Man rührt ca. 30 min bis zur vollständigen Abspaltung der BOC-Gruppe. Anschließend dampft man die Trifluoressigsäure im Vakuum ab, versetzt den Rückstand mit 5 ml Ethylacetat, filtriert von Ungelöstem ab und dampft das Filtrat-zur Trockene ein.
Ausbeute: 0,96 g (98 %)
DC: Kieselgel 60, Eluens Ethylacetat/Methanol = 2:8, RF = 0,2

Beispiel 5

**Synthese von S-Acetyl-mercaptopropionsäure**

Zu 10,6 g (100 mmol) Mercaptopropionsäure wird bei 20°C langsam 8,6 g (110 mmol) Acetylchlorid zugetropft. Nach beendeter Zugabe wird 10 min auf 100°C erhitzt. Das Reaktionsgemisch wird einer Vakuumdestillation unterzogen und das Produkt bei 0,4 bar und 105°C rein gewonnen.
Ausbeute: 5,8 g (36 %)
1-H-NMR ($CDCl_3$): δ (ppm) = 2,3 (s, 3H), 2,7 (t, 2H), 3,1 (t, 2H).

Beispiel 6

**Synthese von N-Succinimidyl-S-acetylthiopropionat, (SATP)**

16,2 g (0,1 mol) S-Acetyl-mercaptopropionsäure, 12,7 g (0,11 mol) N-Hydroxysuccinimid und 22,7 g (0,11 mol) Dicyclohexylcarbodiimid werden in 0,4 l absolutem Ethylacetat 16 h bei 20°C gerührt. Ausgefallener Niederschlag wird abfiltriert und das Filtrat im Vakuum eingedampft. Der ölige Rückstand wird in wenig Ethylacetat aufgenommen und gekühlt. Dabei fällt weiterer Niederschlag aus, der verworfen wird. Dieser Vorgang wird zweimal wiederholt. Aus dem letzten Filtrat erhält man nach Eindampfen 13 g (50 %) SATP.

1H-NMR ($CDCl_3$): δ (ppm) = 2,3 (s, 3H), 2,8 (s, 4H), 2,9 (m, 2H), 3,1 (m, 2H).

Beispiel 7

**Synthese von Biotin-aminocapronsäure-amidodioxaoctyl-mercaptopropionsäure-amid, (Verbindung 1)**

Eine Lösung aus 0,96 g (2 mmol) Biotin-X-DADOO (aus Beispiel 4) und 0,5 g (2 mmol) SATP (aus Beispiel 6) werden in 20 ml Dioxan und 20 ml 0,1 mol/l Kaliumphosphatpuffer pH 8,5 bei 20°C 2 h gerührt. Anschließend wird die Lösung zur Trockene eingedampft, der Rückstand mit 2 ml Trifluoressigsäure aufgenommen und unter Inertgas 0,5 h bei 20°C gerührt. Die Aufreinigung erfolgt durch Flash-Chromatographie an Kieselgel (Eluens: Ethylacetat/Methanol = 3:7).
Ausbeute: 150 mg (13 %)
DC: Kieselgel 60, Ethylacetat/Methanol = 3/7,
RF = 0,35

Beispiel 8

**Synthese von Biotinamido-3,6-dioxaoctyl-S-acetyl-mercaptopropionsäureamid, (Biotin-DADOO-SATP)**

1 g (2,7 mmol) Biotin-DADOO gelöst in 40 ml 0,1 mol/l Kaliumphosphatpuffer pH 7,0 wird langsam mit einer Lösung aus 1,4 g (5,35 mmol) SATP (aus Beispiel 6) in 40 ml Dioxan versetzt. Während der Zugabe muß der pH-Wert kontinuierlich mit 0,1 mol/l Kaliumphosphatpuffer auf pH 7,0 nachgestellt werden. Nach beendeter Zugabe wird noch 10 min gerührt und anschliessend zur Trockene eingeengt. Das Rohprodukt kann ohne weitere Aufreinigung in die folgende Stufe eingesetzt werden.
DC: Kieselgel 60, Eluens Ethylacetat/Methanol = 3,5/7,5,
RF = 0,35.

Beispiel 9

**Synthese von Bis-(biotinamido-3,6-dioxaoctyl)-mercaptopropionsäureamid-disulfid (Verbindung 2)**

1,6 g des Rohproduktes aus Beispiel 8 wird in 100 ml Stickstoff-gesättigtem 0,5 mol/l Kaliumphosphatpuffer pH 7,5 gelöst und mit 5,4 ml 1 mol/l methanolischer Hydroxylamin-Lösung versetzt. Es wird 2 h bei 20°C gerührt, anschließend im Vakuum zur Trockene eingedampft und über Flash-Chromatographie an Kieselgel (Ethylacetat/Methanol = 3/7) aufgereinigt.
Ausbeute: 150 mg (6 %)
DC: Kieselgel 60, Eluens Ethylacetat/Methanol = 3/7, RF = 0,35

Beispiel 10

**Synthese von Biotinamido-3,6-dioxaoctyl-S-acetylmercaptoessigsäureamid, (Biotin-DADOO-SATA)**

Die Herstellung erfolgt analog Beispiel 8 aus 187 mg (0,8 mmol) N-Succinimidyl-S-acetyl-thioacetat (SATA) (Boehringer Mannheim, Best.Nr. 1081765) und 300 mg (0,8 mmol) Biotin-DADOO.
Ausbeute: 109 mg (49 %)
DC: Kieselgel 60, Eluens Ethylacetat/Methanol = 6,5/3,5
RF = 0,35

Beispiel 11

**Synthese von Bis-(biotinamido-3,6-dioxaoctyl)-mercaptoessigsäureamid-disulfid (Verbindung 3)**

Die Herstellung erfolgt analog Beispiel 9 aus 100 mg (0,2 mmol) Biotin-DADOO-SATA und 0,25 ml 1 mol/l methanolischer Hydroxylaminlösung.
Ausbeute: 55 mg (60 %).
DC: Kieselgel 60, Eluens Ethylacetat/Methanol = 3/7,
RF = 0,35

**Beispiel 12**

a) 2-(S-Acetyl)mercaptopropionsäure-[2-(2-Hydroxyethoxy)]-ethylamid

Zu einer Lösung aus 2,14 g (20 mmol) 2-(2-Aminoethoxy)-ethanol in 25 ml THF wurde innerhalb von 15 min eine Lösung aus 5 g (20 mmol) N-Succinimidyl-S-acetylthiopropionat (SATP, Beispiel 2c) in 50 ml THF zugetropft und 2 Std. bei 20°C gerührt. Nach Abschluß der Reaktion (DC-Kontrolle) wurde das Reaktionsgemisch im Vakuum eingedampft und über Kieselgel chromatographiert (Kieselgel 60, Eluens Ethylacetat/Methanol = 7/3 plus 1 % Essigsäure).

DC: (Kieselgel 60, Eluens Ethylacetat/Methanol = 7/3 plus 1 % Essigsäure)
$R_F$ = 0,67.
Ausbeute; 2,7 g.
MS (pos FAB): $MH^+$ = 236.

b) 2-Mercaptopropionsäure-[2-(2-hydroxyethoxy)]-ethylamid (Verbindung 4)

2,7 g (8,7 mmol) der Verbindung a) wurden mit 600 ml 1 mol/l Hydroxylamin-Lösung in Methanol versetzt und 1 Std. bei 20°C gerührt. Anschließend wurde das Lösungsmittel im Vakuum abgezogen und der Rückstand 3 x mit Dichlormethan extrahiert. Man erhielt 1,5 g öliges Rohprodukt, das durch Flashchromatographie an Kieselgel aufgereinigt wurde (Kieselgel 60, Eluens Dichlormethan/Methanol = 9.3/0.7).
DC: (Kieselgel 60, Eluens Dichlormethan/Methanol = 9/1)
$R_F$ = 0,45.
Ausbeute: 0,86 g (farbloses Öl).
MS (pos FAB): $MH^+$ = 194.

Beispiel 13

**Herstellung und Charakterisierung von Monoschichten**

**a) Probenpräparation:**

Die Gold-Substrate wurden durch Bedampfen von Glasobjektträgern aus LASFN 9 (Berliner Glas KG) mit ca. 50 nm Au (99,99%) hergestellt.

Die Bedampfung erfolgte in einer Bedampfungsanlage BAE 250 von Balzers und wurde bei einem Druck von $\leq 5x10^{-6}$ mbar durchgeführt.

**b) Monoschicht-Bildung:**

Die Gold-Substrate wurden direkt nach dem Öffnen der Bedampfungsanlage unter Argon Schutzgas in 0,5 mM Lösungen der Verbindungen in Wasser (MilliQ) gestellt. Nach 6 h Adsorptionszeit wurden die Substrate mit 300 ml Wasser gespült und in einem Stickstoffstrom getrocknet.

**c) Charakterisierung:**

Die Charakterisierung der Monoschicht erfolgte mit Hilfe der Oberflächenplasmonen-Spektroskopie und der Methode der Kontaktwinkelmessung.

Oberflächenplasmonen-Spektroskopie ist eine optische Methode mit hoher Empfindlichkeit zur Charakterisierung von Oberflächen und dünnen Filmen, ohne daß ein spezieller Molekül-Label (z.B. Fluoreszenz-Label) benötigt wird (W. Knoll, MRS Bulletin, Vol. 16, No. 7, 1991, 29-39).

Zur Bestimmung der Schichtdicken der selbst assemblierenden Monoschicht (SAM) wurden Messungen gegen Luft und wäßriges Medium durchgeführt.

Kontaktwinkelmessungen sind ein häufig genutztes Verfahren zur Analyse von Grenzflächen. Dabei lassen sich Informationen über das Substrat, die Art und Zusammensetzung der Bedeckung und die Ordnung auf dem Film gewinnen (A. Ullmann, "Introduction to ultrathin organic films", Academic Press, Inc. 1991). Zur Messung der Kontaktwinkel wurde ein Kontaktwinkelmikroskop G1 (Krüss/Hamburg) verwendet. Alle angegebenen Werte aus Tabelle 1 und 2 stellen Mittelwerte dar aus mindestens 6 Messungen an unterschiedlichen Stellen auf dem Träger. Der Fehler beträgt dabei ± 2 Grad.

In den folgenden Experimenten wurden die Benetzungseigenschaften von durch Ultrafiltration (MilliQ) gereinigtem Wasser auf den jeweiligen Filmen gemessen.

Tabelle 1

| Messergebnisse: Gemessene Schichtdicken und Kontaktwinkel | | | | | |
|---|---|---|---|---|---|
| | Dicke d [Å] der SAM | | | | |
| | d gemessen | d berechnet[2] (senkrecht) | d berechnet[2] (30° geneigt) | n[4] | θa[3] [°] |
| Verb. 4 aus Wasser | 9±2 | 13 | 11 | 1.50 | 26±2 |
| Verb. 1 aus Wasser | 33±2 | 38 | 33 | 1.50 | 35±2 |
| Verb. 1 aus Puffer[1] | 33±2 | 38 | 33 | 1.50 | |

[1] Phosphatpuffer 0.05 mol/l, pH 7.0
[2] Dicke der SAM aus Bindungslängen berechnet
[3] θa [°] bestimmt nach A. Ulmann, "Introduction to ultrathin organic films", Academic Press, Inc. 1991.
[4] Brechungsindex n nach Ullmann, supra

Die gemessenen Dicken stimmen gut mit den theoretisch berechneten Werten überein. Dies weist daraufhin, daß es sich jeweils um geordnete, dicht gepackte Monoschichten handelt. Dabei dürften die Moleküle analog zu den von Whitesides et al. (C.D. Bain, G.M. Whitesides, Science 240 (1988) 62; K.L. Prime, G.M. Whitesides, Science 252 (1991) 1164) publizierten Daten über langkettige Alkanthiole ebenfalls um ca. 30° zur Oberflächennormalen geneigt sein.

Auch die Kontaktwinkel stimmen gut mit den von OH- bzw. Biotin-terminierten Alkanthiolen auf polykristallinem Au erhaltenen Werten überein (H. Wolf, Diplomarbeit Universität Mainz 1991). Sie stützen das Bild von geordneten und dicht gepackten Monolayern.

Zur Untersuchung der Streptavidin (SA)-Bindefähigkeit dieser neuen Verbindungsklasse wurden neben der reinen Verbindung 1 auch Mischungen mit Verbindung 4 untersucht. Ebenfalls wurden weitere Proteinschichten durch Adsorption von biotinylierten Fab-Fragmenten von monoklonalen Antikörpern gegen HCG (Fab<HCG>) und HCG aufgebaut.

Streptavidin und Fab wurden als $5x10^{-7}$ mol/l Lösung und HCG (humanes Choriogonadotropin) als 25 µg/ml Lösung in 0,5 mol/l NaCl zugegeben.

Tabelle 2:

| Gemessene Kontaktwinkel θa und Schichtdicken d[Å] | | | | | | |
|---|---|---|---|---|---|---|
| $X_b$ [1] | θ[°] [2] | d(SAM) [6] | d(SA) [3,7] | d(B-Fab) [4,7] | d(HCG) [7] | d(RSA) [5,7] |
| 0 | 26±2 | 9 | 0 | 0 | | 0 |
| 0,1 | 37±2 | 10 | 40 | 31 | 16 | |
| 0,25 | 40±2 | 20 | 41 | 26 | 14 | |
| 0,50 | 39±2 | 24 | 36 | 23 | 12 | |
| 0,75 | 39±2 | 28 | 31 | 10 | 4 | |
| 1,00 | 35±2 | 33 | 23 | 7 | 5 | |

[1] Molenbruch bezogen auf Verbindung 1.
[2] θa [°] bestimmt nach Ullmann, supra
[3] SA = Streptavidin.
[4] B-Fab = biotinyliertes Fab <HCG>.
[5] Durch Rinderserumalbumin (RSA)-Zugabe hätte bei unvollständiger Belegung der Goldoberfläche durch die Verbindungen 1 und 4 eine Adsorption des Proteins an freien Stellen der Goldoberfläche und damit eine Schichtdickenzunahme erfolgen müssen.
[6] Brechungsindex des Films: n = 1,50 (Ullmann, supra)
[7] Brechungsindex des Films: n = 1,45 (H. Morgan, D.M. Taylor, C.D'Silva, Thin Solid Films 209 (1992) 122).

Die Zunahme der Dicke der Thiol-Monoschicht d(SAM) korreliert mit den Werten aus Tabelle 1.

Durch eine Verdünnung der Verbindung 1 an der Goldoberfläche mittels der Verbindung 4 wird eine Optimierung der Streptavidin-Bindefähigkeit erreicht.

Bei vollständiger Belegung der Oberfläche mit Streptavidin beträgt die gemessene Dicke 40 Å und stimmt damit gut mit dem theoretisch erwarteten Wert (R.C. Ebersole, M.D. Word, J.A. Miller, J.R. Moran, J.Am.Chem.Soc.112 (1990) 3239-3241; P.C. Weber, D.H. Ohlendorf, J.J. Wendoloski, F.R. Salemme, Science 242 (1989) 85) überein.

Diese optimierte Streptavidin-Schicht fungiert wiederum als optimale Bindephase für B-Fab <HCG>, die wiederum für den HCG-Nachweis verwendet werden kann.

Beispiel 14

**Herstellung einer regenerierten Bindematrix**

In analoger Weise, wie in Beispiel 7 beschrieben, können Desthiobiotin und Iminobiotin als Desthiobiotin-X-DADOO und Iminobiotin-X-DADOO an SATP gekoppelt werden.

Diese Verbindungen können als Bindematrix eingesetzt werden, wenn die Bindephase regenerierbar sein soll.

Zur Regeneration wird die Festphase bei Raumtemperatur 10 Minuten mit einer Lösung von 2 x $10^{-4}$ mol/l Biotin in 0,5 mol/l NaCl inkubiert. Anschließend wird zweimal mit 0,5 mol/l NaCl gewaschen.

Die Regeneration der Bindephase erfolgt zu über 90 % (Messung über Oberflächenplasmonenresonanz).

## Patentansprüche

**1.** Verfahren zur Herstellung einer Bindematrix, die ein Trägermaterial und einen daran über Ankergruppen adsorbierten Festphasen-Reaktanden, der mit mindestens einem freien Reaktionspartner bindefähig ist, enthält, wobei der Festphasen-Reaktand eine verdünnte und im wesentlichen lateral homogene Bindeschicht auf der Oberfläche des Trägermaterials bildet,
**dadurch gekennzeichnet**,
daß man das Trägermaterial mit einer wäßrigen Rekationslösung inkubiert, die einen über ein kurzkettiges Spacermolekül, welches eine Alkylengruppe der Formel $(CH_2)_n$ mit n = 1 - 6 enthält, mit der Ankergruppe verknüpften Festphasen-Reaktanden und mindestens ein hydrophiles Verdünnungsmolekül enthält, welches ein mit Ankergruppen versehenes Molekül ist, an das kein Festphasenreaktand gebunden ist.

**2.** Verfahren nach Anspruch 1,
**dadurch gekennzeichnet**,
daß die Belegung des Festphasen-Reaktanden auf der Oberfläche des Trägermaterials von 0,1 bis 90 mol-% der Gesamtbelegung aus Festphasen-Reaktand und Verdünnungsmolekül ist.

3. Verfahren nach Anspruch 2,
**dadurch gekennzeichnet**,
daß die Belegung des Festphasen-Reaktanden auf der Oberfläche des Trägermaterials von 0,5 bis 70 mol-% der Gesamtbelegung ist.

4. Verfahren nach Anspruch 3,
**dadurch gekennzeichnet**,
daß die Belegung des Festphasen-Reaktanden auf der Oberfläche des Trägermaterials von 1 bis 40 % der Gesamtbelegung ist.

5. Verfahren nach einem der Ansprüche 1 bis 4,
**dadurch gekennzeichnet**,
daß das Trägermaterial eine Metall- oder Metalloxidoberfläche aufweist.

6. Verfahren nach Anspruch 5,
**dadurch gekennzeichnet**,
daß das Trägermaterial eine Gold-, Silber- oder Palladiumoberfläche aufweist und die Ankergruppe eine Thiol-, Disulfid- oder eine Phosphingruppe ist.

7. Verfahren nach einem der Ansprüche 1 bis 6,
**dadurch gekennzeichnet**,
daß man einen Festphasen-Reaktanden verwendet, der über ein flexibles Spacermolekül mit der Ankergruppe verknüpft ist.

8. Verfahren nach einem der vorhergehenden Ansprüche,
**dadurch gekennzeichnet**,
daß man eine wäßrige Reaktionslösung verwendet, die frei von organischen Lösungsmitteln und Detergentien ist.

9. Verfahren nach einem der vorhergehenden Ansprüche,
**dadurch gekennzeichnet**,
daß sich zwischen dem Spacermolekül und dem Festphasen-Reaktanden eine hydrophile Linkergruppe befindet.

10. Verfahren nach Anspruch 9,
**dadurch gekennzeichnet**,
daß die hydrophile Linkergruppe eine oder mehrere Oxyethylengruppen enthält.

11. Verfahren nach Anspruch 9,
**dadurch gekennzeichnet**,
daß die hydrophile Linkergruppe durch ein Amin- oder Hydroxyl-terminiertes Polyethylenoxid gebildet wird.

12. Verfahren nach Anspruch 9,
**dadurch gekennzeichnet**,
daß die hydrophile Linkergruppe aus 1,8-Diamino-3,6-dioxaoktan gebildet ist.

13. Verfahren nach einem der vorhergehenden Ansprüche,
**dadurch gekennzeichnet**,
daß sich zwischen der hydrophilen Linkergruppe und dem Festphasen-Reaktanden noch ein weiteres Spacermolekül befindet.

14. Verfahren nach einem der vorhergehenden Ansprüche,
**dadurch gekennzeichnet**,
daß das hydrophile Verdünnungsmolekül eine Ankergruppe und einen Spacerbestandteil enthält.

15. Verfahren nach Anspruch 14,
**dadurch gekennzeichnet**,
daß das hydrophile Verdünnungsmolekül zusätzlich eine hydrophile Linkergruppe enthält.

16. Verfahren nach Anspruch 14 oder 15,

**dadurch gekennzeichnet**,
daß man als Verdünnungsmolekül eine Verbindung der allgemeinen Formeln

$$X^1 - S - S - X^2 \text{ oder } HS - X^3$$

verwendet, worin $X^1$, $X^2$ und $X^3$ jeweils

$$-(CH_2)_n-\overset{O}{\overset{\|}{C}}-NH-L-Y$$

bedeuten, worin

- n eine natürliche Zahl von 1 bis 6 bedeutet,
- L eine hydrophile Linkergruppe mit einer Kettenlänge von 4 bis 15 Atomen ist und
- Y eine hydrophile Endgruppe ist.

**17.** Verfahren nach Anspruch 16,
**dadurch gekennzeichnet**,
daß die hydrophile Endegruppe $-NH_2-$, -OH, -COOH oder $-SO_3H$ ist.

**18.** Verfahren nach einem der Ansprüche 15 bis 17,
**dadurch gekennzeichnet**,
daß die hydrophile Linkergruppe eine oder mehrere Oxyethylengruppen enthält.

**19.** Verfahren nach einem der vorhergehenden Ansprüche,
**dadurch gekennzeichnet**,
daß man 2-Mercaptopropionsäure[2-(2-hydroxyethoxy)]-ethylamid als Verdünnungsmolekül verwendet.

**20.** Verfahren nach einem der vorhergehenden Ansprüche,
**dadurch gekennzeichnet**,
daß der Festphasen-Reaktand ein mit einem Antikörper bindefähiges Antigen oder Hapten ist.

**21.** Verfahren nach einem der Ansprüche 1 bis 19,
**dadurch gekennzeichnet**,
daß der Festphasen-Reaktand Biotin oder ein Biotinderivat ist.

**22.** Verfahren nach Anspruch 21,
**dadurch gekennzeichnet**,
daß der Festphasen-Reaktand ein Biotinderivat mit einer im Vergleich zu.Biotin geringeren Bindeaffinität an Streptavidin ist.

**23.** Verfahren nach Anspruch 21 oder 22,
**dadurch gekennzeichnet**,
daß der mit dem Festphasen-Reaktanden bindefähige freie Reaktionspartner Streptavidin, Avidin oder ein Derivat davon ist.

**24.** Verfahren nach einem der vorhergehenden Ansprüche,
**dadurch gekennzeichnet,**
daß man nach Adsorption des mit Ankergruppen versehenen Festphasen-Reaktanden an das Trägermaterial die resultierende Bindematrix mit einer oder mehreren weiteren, mit der Bindematrix bindefähigen Substanzen inkubiert, um einen aus mehreren, nicht-kovalent verknüpften Komponenten bestehenden Festphasen-Reaktanden zu erzeugen.

**25.** Verfahren zur Bestimmung eines Analyten in einer Probelösung mittels einer spezifischen Bindungsreaktion zwischen mindestens zwei bioaffinen Reaktanden, von denen einer an eine Festphase gekuppelt vorliegt und der oder die anderen Reaktionspartner frei sind,
**dadurch gekennzeichnet,**
daß man einen Festphasen-Reaktanden verwendet, der Bestandteil einer Bindematrix ist, die nach einem der Ansprüche 1 bis 24 hergestellt worden ist.

**26.** Verfahren nach Anspruch 25,
**dadurch gekennzeichnet,**
daß man die spezifische Bindungsreaktion optisch, elektronisch, über die Wärmetönung oder die Massenbilanz bestimmt.

**27.** Verfahren nach Anspruch 25,
**dadurch gekennzeichnet,**
daß man die spezifische Bindungsreaktion durch reflexionsoptische Techniken bestimmt.

**28.** Verfahren nach Anspruch 27,
**dadurch gekennzeichnet,**
daß man die spezifische Bindungsreaktion plasmonenspektroskopisch bestimmt.

**29.** Verfahren nach Anspruch 25,
**dadurch gekennzeichnet**,
daß man die spezifische Bindungsreaktion potentiometrisch oder amperometrisch bestimmt.

**30.** Verfahren nach Anspruch 25,
**dadurch gekennzeichnet**,
daß man die spezifische Bindungsreaktion über die elektrische Leitfähigkeit oder Kapazitätsänderung bestimmt.

**31.** Verfahren nach einem der Ansprüche 25 bis 30,
**dadurch gekennzeichnet**,
daß man die Bindematrix nach Durchführung einer Analytenbestimmung regeneriert, indem der an den Festphasen-Reaktanden gebundene freie Reaktionspartner durch Zugabe eines weiteren freien Reaktanden von der Bindematrix abgelöst wird.

**32.** Verfahren nach Anspruch 31,
**dadurch gekennzeichnet**,
daß der weitere freie Reaktand eine höhere Affinität gegenüber dem Reaktionspartner als der Festphasen-Reaktand besitzt.

**33.** Verwendung einer Bindematrix, die nach einem der Ansprüche 1 bis 24 hergestellt worden ist, als regenerierbare Schicht.

**34.** Verbindung der allgemeinen Formeln

$$X^1 - S - S - X^2 \text{ oder } HS - X^3$$

worin
$X^1$, $X^2$ und $X^3$ jeweils

$$-(CH_2)_n-\overset{\overset{\displaystyle O}{\|}}{C}-NH-L-Y$$

bedeuten, worin

n eine natürliche Zahl von 1 bis 6 bedeutet,

L eine hydrophile Linkergruppe mit einer Kettenlänge von 4 bis 15 Atomen ist und
Y eine hydrophile Endgruppe ist.

**35.** Verbindung nach Anspruch 34, **dadurch gekennzeichnet,** daß die hydrophile Endegruppe $-NH_2$, -OH, -COOH oder $-SO_3H$ ist.

**36.** Verbindung nach einem der Ansprüche 34 oder 35, **dadurch gekennzeichnet,** daß die hydrophile Linkergruppe eine oder mehrere Oxyethylengruppen enthält.

**37.** 2-Mercaptopropionsäure[2-(2-hydroxyethoxy)]-ethylamid.

## Claims

**1.** Process for the production of a binding matrix containing a carrier material and a solid phase reactant which is adsorbed to this via anchor groups that is capable of binding to at least one free reaction partner, the said solid phase reactant forming a dilute and essentially laterally homogeneous binding layer on the surface of the carrier material, **wherein** the carrier material is incubated with an aqueous reaction solution which contains a solid phase reactant linked to the anchor group via a short-chain spacer molecule which contains an alkylene group of formula $(CH_2)_n$ in which n = 1 - 6 and contains at least one hydrophilic diluent molecule which is a molecule provided with anchor groups to which no solid phase reactant is bound.

**2.** Process as claimed in claim 1, **wherein** the coverage of the solid phase reactant on the surface of the carrier material is 0.1 to 90 mol % of the total coverage of the solid phase reactant and diluent molecule.

**3.** Process as claimed in claim 2, **wherein** the coverage of the solid phase reactant on the surface of the carrier material is 0.5 to 70 mol % of the total coverage.

**4.** Process as claimed in claim 3, **wherein** the coverage of the solid phase reactant on the surface of the carrier material is 1 to 40 % of the total coverage.

**5.** Process as claimed in one of the claims 1 to 4, **wherein** the carrier material has a metal or metal oxide surface.

**6.** Process as claimed in claim 5, **wherein** the carrier material has a gold, silver or palladium surface and the anchor group is a thiol group, disulfide group or a phosphine group.

**7.** Process as claimed in one of the claims 1 to 6, **wherein** a solid phase reactant is used which is linked to the anchor group via a flexible spacer molecule.

**8.** Process as claimed in one of the previous claims, **wherein** an aqueous reaction solution is used which is free of organic solvents and detergents.

**9.** Process as claimed in one of the previous claims, **wherein**

a hydrophilic linker group is located between the spacer molecule and the solid phase reactant.

**10.** Process as claimed in claim 9,
**wherein**
the hydrophilic linker group contains one or several oxyethylene groups.

**11.** Process as claimed in claim 9,
**wherein**
the hydrophilic linker group is formed by an amino-terminated or hydroxyl-terminated polyethylene oxide.

**12.** Process as claimed in claim 9,
**wherein**
the hydrophilic linker group is formed from 1,8-diamino-3,6-dioxaoctane.

**13.** Process as claimed in one of the previous claims,
**wherein**
a further spacer molecule is located between the hydrophilic linker group and the solid phase reactant.

**14.** Process as claimed in one of the previous claims,
**wherein**
the hydrophilic diluent molecule contains an anchor group and a spacer component.

**15.** Process as claimed in claim 14,
**wherein**
the hydrophilic diluent molecule additionally contains a hydrophilic linker group.

**16.** Process as claimed in claim 14 or 15,
**wherein**
a compound of the general formulae

$$X^1 - S - S - X^2 \text{ or } HS - X^3$$

is used as the diluent molecule in which
$X^1$, $X^2$ and $X^3$ each denote

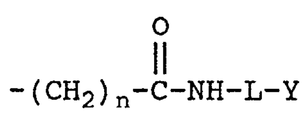

in which

n denotes a natural number from 1 to 6
L is a hydrophilic linker group with a chain length of 4 to 15 atoms and
Y is a hydrophilic terminal group.

**17.** Process as claimed in claim 16,
**wherein**
the hydrophilic terminal group is $-NH_2$, -OH, -COOH or $-SO_3H$.

**18.** Process as claimed in one of the claims 15 to 17,
**wherein**
the hydrophilic linker group contains one or several oxyethylene groups.

**19.** Process as claimed in one of the previous claims,

**wherein**
2-mercaptopropionic acid[2-(2-hydroxyethoxy)]-ethylamide is used as the diluent molecule.

**20.** Process as claimed in one of the previous claims,
**wherein**
the solid phase reactant is an antigen or hapten capable of binding to an antibody.

**21.** Process as claimed in one of the claims 1 to 19,
**wherein**
the solid phase reactant is biotin or a biotin derivative.

**22.** Process as claimed in claim 21,
**wherein**
the solid phase reactant is a biotin derivative with a lower binding affinity to streptavidin compared to biotin.

**23.** Process as claimed in claim 21 or 22,
**wherein**
the free reaction partner capable of binding to the solid phase reactant is streptavidin, avidin or a derivative thereof.

**24.** Process as claimed in one of the previous claims,
**wherein**
after adsorption of the solid phase reactant provided with anchor groups to the carrier material, the resulting binding matrix is incubated with one or several further substances that can bind to the binding matrix in order to produce a solid phase reactant composed of several non-covalently linked components.

**25.** Method for the determination of an analyte in a sample solution by means of a specific binding reaction between at least two reactants with bioaffinity one of which is present coupled to a solid phase and the other reaction partner or reaction partners are free,
**wherein**
a solid phase reactant is used which is a component of a binding matrix which has been produced as claimed in one of the claims 1 to 24.

**26.** Method as claimed in claim 25,
**wherein**
the specific binding reaction is determined optically, electronically, via heat tonality or mass analysis.

**27.** Method as claimed in claim 25,
**wherein**
the specific binding reaction is determined by optical reflection techniques.

**28.** Method as claimed in claim 27,
**wherein**
the specific binding reaction is determind by plasmon spectroscopy.

**29.** Method as claimed in claim 25,
**wherein**
the specific binding reaction is determined potentiometrically or amperometrically.

**30.** Method as claimed in claim 25,
**wherein**
the specific binding reaction is determined by means of the electrical conductivity or change in capacitance.

**31.** Process as claimed in one of the claims 25 to 30,
**wherein**,
after determining an analyte the binding matrix is regenerated by detaching the free reaction partner bound to the solid phase reactant from the binding matrix by adding a further free reactant.

**32.** Process as claimed in claim 31,

**wherein**,
the further free reactant has a higher affinity towards the reaction partner than that of the solid phase reactant.

**33.** Use of a binding matrix produced according to one of the claims 1 to 24 as a layer that can be regenerated.

**34.** Compound of the general formulae

$$X^1 - S - S - X^2 \text{ or } HS - X^3$$

in which
$X^1$, $X^2$ and $X^3$ each denote

$$-(CH_2)_n-\overset{\overset{O}{\|}}{C}-NH-L-Y$$

in which

n denotes a natural number from 1 to 6
L is a hydrophilic linker group with a chain length of 4 to 15 atoms and
Y is a hydrophilic terminal group.

**35.** Compound as claimed in claim 34,
**wherein**
the hydrophilic terminal group is $-NH_2$, -OH, -COOH or $-SO_3H$.

**36.** Compound as claimed in claims 34 or 35,
**wherein**
the hydrophilic linker group contains one or several oxyethylene groups.

**37.** 2-Mercaptopropionic acid[2-(2-hydroxyethoxy)]-ethylamide.

**Revendications**

**1.** Procédé de préparation d'une matrice de liaison qui contient un matériau support et un corps réagissant en phase solide adsorbé sur celle-ci par l'intermédiaire de groupes d'ancrage, qui est capable de se lier avec au moins un partenaire réactionnel libre, le corps réagissant en phase solide formant sur la surface du matériau support une couche de liaison diluée et sensiblement homogène latéralement, caractérisé en ce que l'on incube le matériau support avec une solution réactionnelle aqueuse qui contient un corps réagissant en phase solide lié au groupe d'ancrage par l'intermédiaire d'une molécule espaceur à chaîne courte qui contient un groupe alkylène de formule $(CH_2)_n$ avec n = 1 - 6 et au moins une molécule de dilution hydrophile qui est une molécule munie de groupes d'ancrage à laquelle aucun corps réagissant en phase solide n'est lié.

**2.** Procédé selon la revendication 1 caractérisé en ce que la couverture de la surface du matériau support par le corps réagissant en phase solide est de 0,1 à 90 mol% de la couverture totale par le corps réagissant en phase solide et la molécule de dilution.

**3.** Procédé selon la revendication 2 caractérisé en ce que la couverture de la surface du matériau support par le corps réagissant en phase solide est de 0,5 à 70 mol% de la couverture totale.

**4.** Procédé selon la revendication 3 caractérisé en ce que la couverture de la surface du matériau support par le corps réagissant en phase solide est de 1 à 40% de la couverture totale.

**5.** Procédé selon l'une des revendications 1 à 4 caractérisé en ce que le matériau support comporte une surface métallique ou d'oxyde métallique.

**6.** Procédé selon la revendication 5 caractérisé en ce que le matériau support comporte une surface d'or, d'argent ou de palladium et le groupe d'ancrage est un groupe thiol, disulfure ou phosphine.

**7.** Procédé selon l'une des revendications 1 à 6 caractérisé en ce que l'on utilise un corps réagissant en phase solide qui est lié au groupe d'ancrage par l'intermédiaire d'une molécule espaceur flexible.

**8.** Procédé selon l'une des revendications précédentes caractérisé en ce que l'on utilise une solution réactionnelle aqueuse qui est exempte de solvants organiques et de détergents.

**9.** Procédé selon l'une des revendications précédentes caractérisé en ce qu'un groupe lieur hydrophile est présent entre la molécule espaceur et le corps réagissant en phase solide.

**10.** Procédé selon la revendication 9 caractérisé en ce que le groupe lieur hydrophile contient un ou plusieurs groupes oxyéthylène.

**11.** Procédé selon la revendication 9 caractérisé en ce que le groupe lieur hydrophile est formé par un poly(oxyde d'éthylène) terminé par amine ou hydroxyle.

**12.** Procédé selon la revendication 9 caractérisé en ce que le groupe lieur hydrophile est formé par le 1,8-diamino-3,6-dioxaoctane.

**13.** Procédé selon l'une des revendications précédentes caractérisé en ce qu'une autre molécule espaceur encore se trouve entre le groupe lieur hydrophile et le corps réagissant en phase solide.

**14.** Procédé selon l'une des revendications précédentes caractérisé en ce que la molécule de dilution hydrophile contient un groupe d'ancrage et une partie espaceur.

**15.** Procédé selon la revendication 14 caractérisé en ce que la molécule de dilution hydrophile contient en outre un groupe lieur hydrophile.

**16.** Procédé selon la revendication 14 ou 15 caractérisé en ce que l'on utilise comme molécule de dilution un composé de formule générale

$$X^1 - S - S - X^2 \text{ ou } HS - X^3$$

où
$X^1$, $X^2$ et $X^3$ représentent chacun

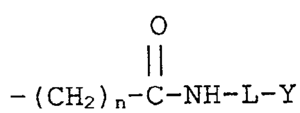

où

n représente un nombre naturel de 1 à 6,
L est un groupe lieur hydrophile ayant une longueur de chaîne de 4 à 15 atomes et
Y est un groupe terminal hydrophile.

**17.** Procédé selon la revendication 16 caractérisé en ce que le groupe terminal hydrophile est $-NH_2$, -OH, -COOH ou $-SO_3H$.

**18.** Procédé selon l'une des revendications 15 à 17 caractérisé en ce que le groupe lieur hydrophile contient un ou plusieurs groupes oxyéthylène.

**19.** Procédé selon l'une des revendications précédentes caractérisé en ce que l'on utilise le [2-(2-hydroxyéthoxy)] éthylamide de l'acide 2-mercaptopropionique comme molécule de dilution.

**20.** Procédé selon l'une des revendications précédentes caractérisé en ce que le corps réagissant en phase solide est un antigène ou haptène capable de se lier avec un anticorps.

**21.** Procédé selon l'une des revendications 1 à 19 caractérisé en ce que le corps réagissant en phase solide est la biotine ou un dérivé de la biotine.

**22.** Procédé selon la revendication 21 caractérisé en ce que le corps réagissant en phase solide est un dérivé de la biotine ayant, par comparaison avec la biotine, une plus faible affinité de liaison avec la streptavidine.

**23.** Procédé selon la revendication 21 ou 22 caractérisé en ce que le partenaire réactionnel libre capable de se lier avec le corps réagissant en phase solide est la streptavidine, l'avidine ou un dérivé de celles-ci.

**24.** Procédé selon l'une des revendications précédentes caractérisé en ce que, après adsorption du corps réagissant en phase solide muni de groupes d'ancrage sur le matériau support, on incube la matrice de liaison résultante avec une ou plusieurs autres substances capables de se lier avec la matrice de liaison pour produire un corps réagissant en phase solide consistant en plusieurs constituants liés de manière non covalente.

**25.** Procédé pour déterminer un analyte dans une solution échantillon au moyen d'une réaction de liaison spécifique entre au moins deux corps réagissants à bioaffinité, parmi lesquels l'un est présent couplé à une phase solide et l'autre ou les autres partenaires réactionnels sont libres, caractérisé en ce que l'on utilise un corps réagissant en phase solide qui fait partie d'une matrice de liaison qui a été préparée selon l'une des revendications 1 à 24.

**26.** Procédé selon la revendication 25 caractérisé en ce que l'on détermine la réaction de liaison spécifique par voie optique, électronique, par le biais de la chaleur de réaction ou du bilan de masse.

**27.** Procédé selon la revendication 25 caractérisé en ce que l'on détermine la réaction de liaison spécifique par des techniques optiques de réflexion.

**28.** Procédé selon la revendication 27 caractérisé en ce que l'on détermine la réaction de liaison spécifique par spectroscopie plasmonique.

**29.** Procédé selon la revendication 25 caractérisé en ce que l'on détermine la réaction de liaison spécifique par potentiométrie ou par ampérométrie.

**30.** Procédé selon la revendication 25 caractérisé en ce que l'on détermine la réaction de liaison spécifique par le biais de la conductivité électrique ou d'une variation de capacité.

**31.** Procédé selon l'une des revendications 25 à 30 caractérisé en ce que l'on régénère la matrice de liaison après la mise en oeuvre d'une détermination d'analyte par le fait que le partenaire réactionnel libre lié avec le corps réagissant en phase solide est séparé de la matrice de liaison par addition d'un autre corps réagissant libre.

**32.** Procédé selon la revendication 31 caractérisé en ce que l'autre corps réagissant libre possède une affinité pour le partenaire réactionnel plus élevée que le corps réagissant en phase solide.

**33.** Utilisation comme couche régénérable d'une matrice de liaison qui a été préparée selon l'une des revendications 1 à 24.

**34.** Composé de formule générale

$$X^1 - S - S - X^2 \text{ ou } HS - X^3$$

où
$X^1$, $X^2$ et $X^3$ représentent chacun

$$-(CH_2)_n-\overset{\overset{O}{||}}{C}-NH-L-Y$$

où

n représente un nombre naturel de 1 à 6,
L est un groupe lieur hydrophile ayant une longueur de chaîne de 4 à 15 atomes et
Y est un groupe terminal hydrophile.

**35.** Composé selon la revendication 34 caractérisé en ce que le groupe terminal hydrophile est $-NH_2$, -OH, -COOH ou $-SO_3H$.

**36.** Composé selon l'une des revendications 34 et 35 caractérisé en ce que le groupe lieur hydrophile contient un ou plusieurs groupes oxyéthylène.

**37.** [2-(2-hydroxyéthoxy)]éthylamide de l'acide 2-mercaptopropionique.

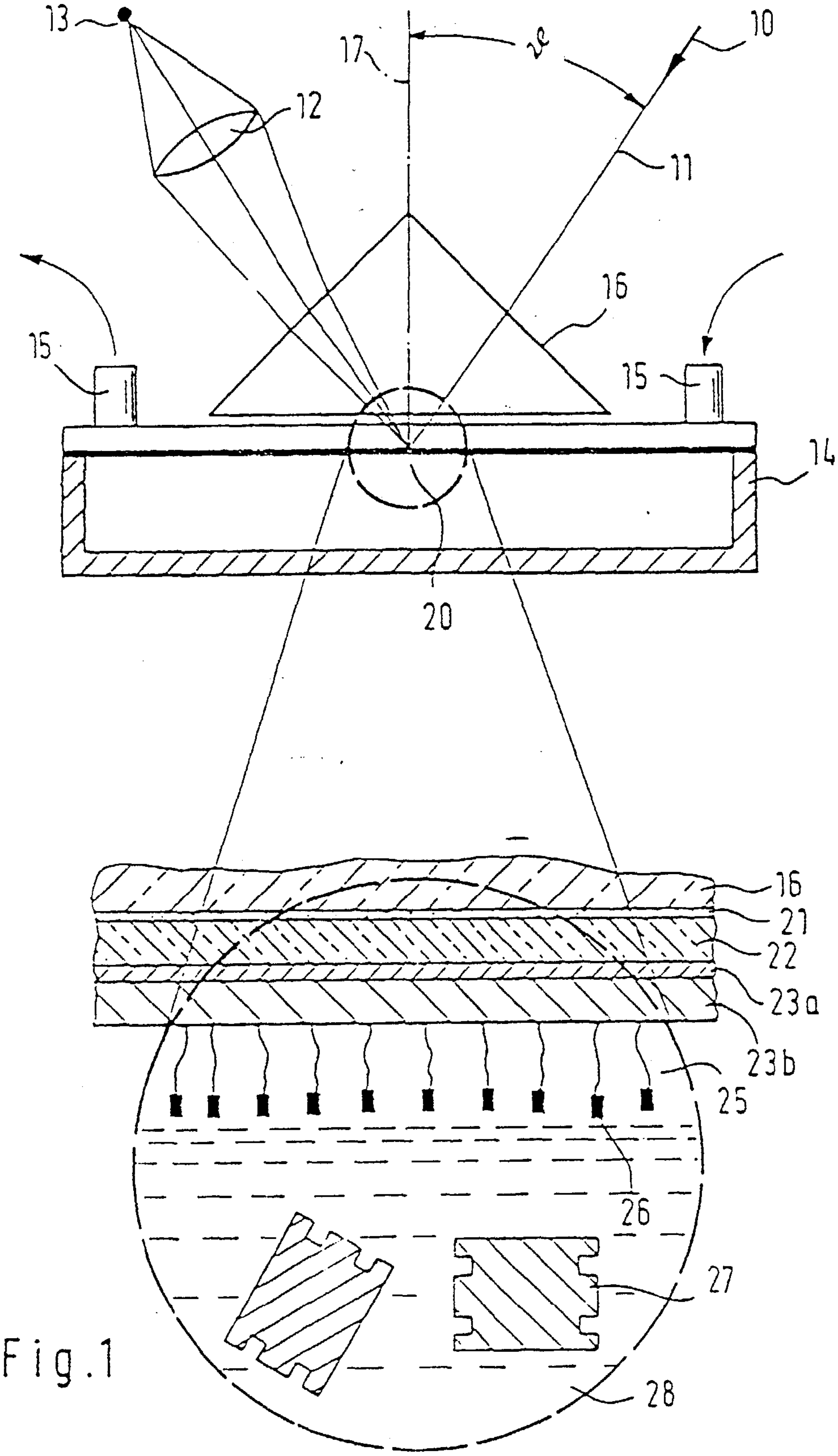


Fig.1

Figur 2

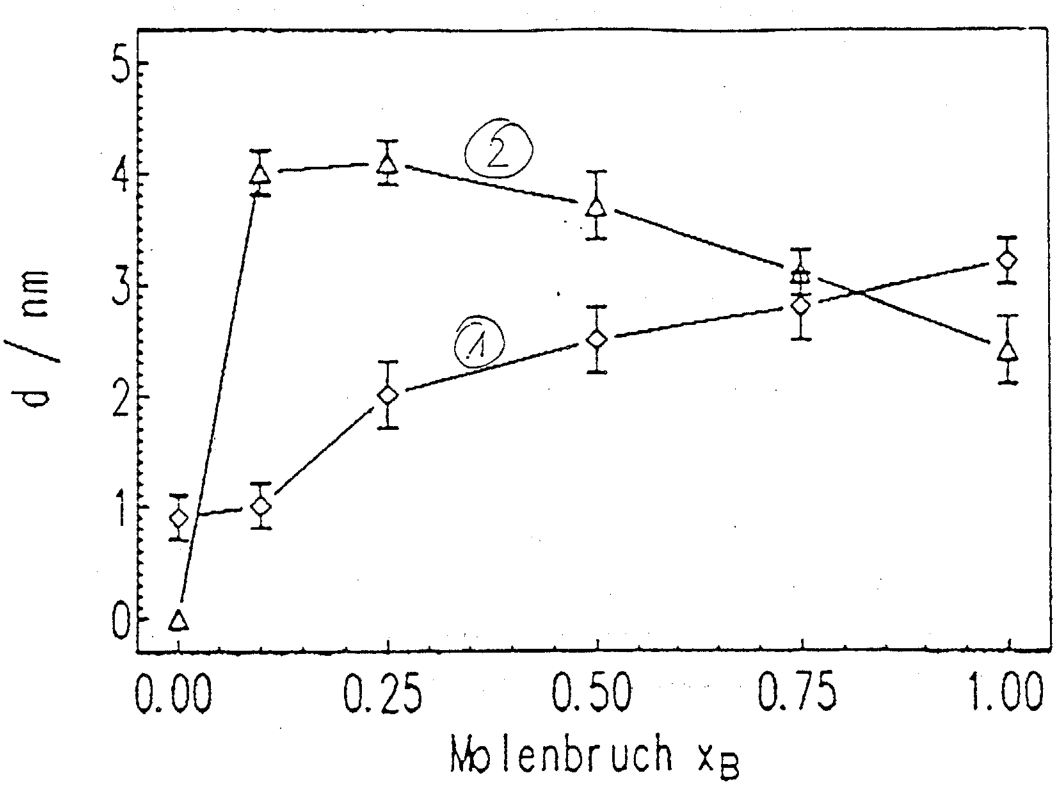

Figur 3

Verbindung 1:

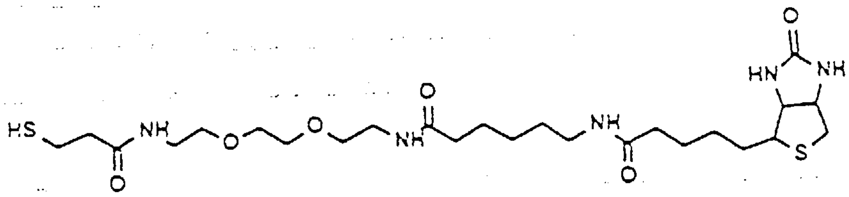

Verbindung 2:

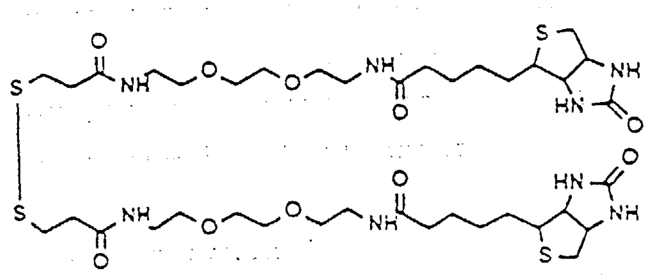

Verbindung 3:

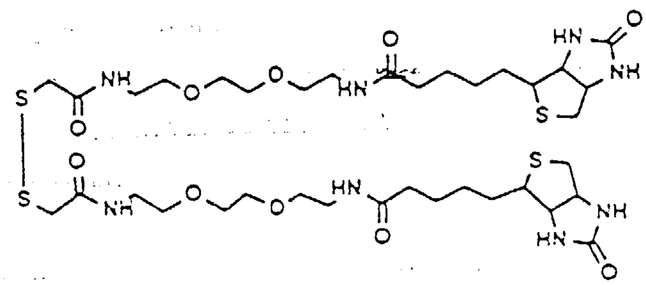

Verbindung 4: